(19) **Europäisches Patentamt European Patent Office Office européen des brevets**

(11) **EP 1 726 288 B1**

(12) **EUROPEAN PATENT SPECIFICATION**

(45) Date of publication and mention
of the grant of the patent:
**16.11.2016 Bulletin 2016/46**

(21) Application number: **05727074.6**

(22) Date of filing: **22.03.2005**

(51) Int Cl.:
*A61K 8/18* *(2006.01)*      *A61Q 11/00* *(2006.01)*
*A61K 8/73* *(2006.01)*

(86) International application number:
**PCT/JP2005/005146**

(87) International publication number:
**WO 2005/089703 (29.09.2005 Gazette 2005/39)**

(54) **COMPOSITION FOR TOOTHBRUSHING**

ZAHNPUTZZUSAMMENSETZUNG

COMPOSITION POUR DENTIFRICE

(84) Designated Contracting States:
**DE FR GB**

(30) Priority: **19.03.2004   JP 2004079679**
**21.05.2004   JP 2004152143**
**03.09.2004   JP 2004257541**
**03.09.2004   JP 2004257542**

(43) Date of publication of application:
**29.11.2006   Bulletin 2006/48**

(60) Divisional application:
**12185405.3 / 2 548 547**

(73) Proprietor: **KAO CORPORATION**
**Tokyo 103-8210 (JP)**

(72) Inventors:
• **YOSHIDA, Hidenori,**
**KAO CORPORATION RESEARCH LAB.**
**Tokyo 1318501 (JP)**
• **MURAKAMI, Yoshinori,**
**KAO CORPORATION RESEARCH LAB.**
**Tokyo 1318501 (JP)**

• **OSHINO, Kazushi,**
**KAO CORPORATION RESEARCH LAB.**
**Tokyo 1318501 (JP)**

(74) Representative: **Hoffmann Eitle**
**Patent- und Rechtsanwälte PartmbB**
**Arabellastraße 30**
**81925 München (DE)**

(56) References cited:
**EP-A1- 0 480 172      WO-A2-2004/071321**
**JP-A- 1 299 211      JP-A- 9 012 436**
**JP-A- 11 171 750      JP-A- 11 171 751**
**JP-A- 11 199 456      JP-A- 55 098 111**
**JP-A- S60 159 721      JP-A- 2001 172 145**
**JP-A- 2004 010 576**

Remarks:
The file contains technical information submitted after the application was filed and not included in this specification

EP 1 726 288 B1

**Description**

[Technical field]

The present invention relates to a dentifrice composition excellent in plaque and stain removing effects and also feeling upon use.

[Background Art]

As a dentifrice containing cellulose therein, a dentifrice composition that contains a cellulose powder having an average particle size of from 0.005 to 1 mm is disclosed as a substitute for an abrasive (Patent Document 1). Such a cellulose powder, however, cannot be said to possess sufficient plaque removing power, so that use of a granulated cellulose having an average particle size of from 50 to 1000 $\mu$m was proposed in lieu of such a cellulose powder (Patent Document 2). Nevertheless, such a granulated cellulose is still not sufficient in color-removing effects, and thus there has been a demand for use of non-granulated cellulose from the viewpoint of its production cost.
From the viewpoints of detergency and feeling upon use, there has also been a demand for a dentifrice composition enabling an adequate amount of foam to last, thereby making it easier to brush the teeth for a long period of time. With regard to foaming, a high foaming property is usually preferred. The foaming property or foaming rate of various surfactants has been investigated conventionally. In order to heighten the foaming property, there is a proposal indicating that the foaming amount should be increased, or a proposal indicating that foams with a high shape retention should be maintained for long hours (Patent Document 3). However, no report has yet been made on the relationship between the foam quality and foaming amount of cellulose powder and a dentifrice composition containing it.
Patent Document 4 discloses a tooth paste composition with reduced abrasivity which includes 0.05 to 3.0 wt.-% of a binder, 0.8 to 3.0 wt.-% of a surfactant, 2 to 22 wt.-% silica and 0.1 to 8.0 wt.-% microcrystalline cellulose.
Patent Document 5 concerns a composition for oral cavity having high cleaning effect and low polishing property containing 0.2-20 wt.% crystalline cellulose powder and 5-60% calcium hydrogen phosphate.
Patent Document 6 relates to a preparation for the care of the teeth which consists of the customary known ingredients of a preparation for the care of the teeth and which contains cellulose powder in place of customary known polishing particles.

[Patent Document 1] JP-A-55-98111
[Patent Document 2] JP-A-09-40537
[Patent Document 3] JP-11-209255
[Patent Document 4] WO 2004/071321 A2
[Patent Document 5] JP-11-199456
[Patent Document 6] EP 0 480 172 A1

[Disclosure of the Invention]

In the present invention, there is thus provided a dentifrice composition containing the following components (A), (B) and (C):

(A) from 0.2 to 3 weight% of powder cellulose having an average polymerization degree of 350 or greater;
(B) a surfactant; and
(C) from 10 to 30 weight% of an abrasive,

wherein the abrasive has an RDA of from 20 to 200.

[Brief Description of the Drawings]

[FIG. 1] FIG. 1 is a schematic view illustrating an interdental model used for a stain removal test and an interdental space to be evaluated.
[FIG. 2] FIG. 2 is a graph showing the results of the stain removal test.
[FIG. 3] FIG. 3 is a schematic view of a brushing machine.

[Mode for Carrying out the Invention]

The present inventors have carried out an extensive investigation to obtain a dentifrice composition excellent in stain removing effect and also in feeling upon use. As a result, it has been found that by using cellulose powder, a surfactant and an abrasive having an RDA of from 20 to 200, a dentifrice composition excellent in stain removing effect during brushing teeth and also excellent in feeling upon use can be obtained.

The dentifrice composition according to one aspect of the present invention contains (A) cellulose powder, (B) a surfactant and (C) an abrasive having an RDA of from 20 to 200.

As the cellulose powder (A) to be used in the present invention, one or more kinds obtained by chemically treating celluloses such as pulp powder, insoluble powder cellulose, powdered α-cellulose and pulp to insolubilize them and then grinding them can be used. From the standing points of stain removal effect, the cellulose powder has preferably an average polymerization degree of 350 or greater, more preferably from about 350 to 2250, still more preferably from about 440 to 2550. From the viewpoints of a fine touch feel and a foam retention, the cellulose powder is preferably a non-granulated cellulose powder having an average particle size of from 10 to 300 μm, more preferably from 10 to 100 μm, still more preferably from 15 to 50 μm.

The content of the cellulose powder in the dentifrice composition is preferably from 0.2 to 3 weight% in the whole composition. From the viewpoints of improving a stain removing effect and preparing viscous foams, the content is preferably 0.2 weight% or greater, more preferably 0.4 weight% or greater. From the viewpoint of the feeling of the dentifrice composition upon use, the content is preferably 3 weight% or greater, more preferably 2 weight% or greater, especially preferably 1 weight% or greater.

The dentifrice composition of the present invention may be prepared by adding the cellulose powder in the powder form or in the form of a dispersion obtained by dispersing the cellulose powder in a liquid such as water, a lower alcohol or polyol. Examples of the lower alcohol include ethanol and isopropanol, while those of the polyol include glycerin, polyethylene glycol and propylene glycol. When it is prepared, use of a dispersion having the cellulose powder dispersed in water, a lower alcohol or a polyol is preferred.

The cellulose powder itself has almost no abrasive power, but small amount of addition considerably enhances the action of an abrasive. This is presumed to occur because owing to the existence of the cellulose powder between bristles and abrasive grains of a teeth brush, the cellulose powder contributes to interaction (friction) between abrasive grains and tooth surface. The cellulose powder is presumed to serve also as a buffer material and exhibits selective detergency to remove stain without much wear of dentin so that it is advantageous. Particularly when an abrasive with low RDA is used, a large amount of an ordinarily used abrasive must be added. Addition of the cellulose powder however can reduce the amount of an abrasive.

The dentifrice composition of the present invention contains a surfactant (B) such as an anionic surfactant, an nonionic surfactant or an amphoteric surfactant. Of these, an anionic surfactant is preferred.

No particular limitation is imposed on the anionic surfactant insofar as it is an anionic surfactant ordinarily used for dentifrice compositions. Examples include acylaminoacid salts such as sodium acylglutamate and acylsalcosin sodium, alkylphosphates such as sodium alkylphosphate, alkyl sulfate ester salts, higher fatty acid sulfonated monoglyceride salts, fatty ace ester salts of isothionic acid, N-methyl(long chain acyl)taurin sodium salts and polyoxyethylene monoalkyl phosphates.

Anionic surfactants having, in the hydrophobic group thereof, an acyl or alkyl group having from 6 to 18 carbon atoms, especially preferably from 10 to 14 carbon atoms are preferred, of which sodium salts are preferred. Alkyl sulfate esters are especially preferred because they are available at a low cost. The anionic surfactant is preferably added in an amount of from 0.1 to 5 weight%, especially preferably from 0.1 to 2 weight% in the dentifrice composition of the present invention.

No particular limitation is imposed on the nonionic surfactant insofar as it is a nonionic surfactant ordinarily used for dentifrice compositions. Examples include polyoxyalkylene addition surfactants, amine-oxide surfactants, mono- or diethanolamide surfactants, sucrose fatty acid esters, glycerin fatty acid esters, polyglycerin fatty acid esters, sorbitan fatty acid esters, polyoxyethylene sorbitan fatty acid esters, and polyoxyethylene hydrogenated castor oils. Of these, sucrose fatty acid esters, glycerin fatty acid esters, polyglycerin fatty acid esters, sorbitan fatty acid esters, polyoxyethylene sorbitan fatty acid esters, polyoxyethylene hydrogenated castor oils are preferred. In the present invention, one or more of them may be used. Nonionic surfactants having an HLB number (Griffin) of 16 or greater, especially from 18 to 20 are preferred.

(Equation 1)

HLB number = 20(1-S/A)

(wherein, S: saponification number, A: acid number of a fatty acid employed.

The fatty acid moiety of these nonionic surfactants has preferably from 6 to 18 carbon atoms. Its content is preferably from 0.1 to 30 wt.%, more preferably from 0.2 to 10 wt.%, still more preferably fro 1.2 to 3.0 wt.%, still more preferably from 1.3 to 2.5 wt.%, especially preferably from 1.3 to 2.0 wt.%. The dentifrice composition preferably does not contain a nonionic surfactant having an HLB number of 15 or less in order to obtain viscous foams. When the nonionic surfactant having an HLB number of 15 or less is added, its amount is preferably 0.5 parts by mass, more preferably 0.3 parts by mass or less, each relative to 1 part by mass of the anionic surfactant.

No particular limitation is imposed on the amphoteric surfactant insofar as it is an amphoteric surfactant ordinarily employed for dentifrice compositions. Examples include alkyl betaine, alkyl amidobetaine, imidazoline and glycine. Its content is preferably from 0.01 to 10 weight%, more preferably from 0.05 to 5 weight% in the whole dentifrice composition.

No particular limitation is imposed on the abrasive (C) to be used in the dentifrice composition of the present invention insofar as it is used ordinarily for dentifrice compositions. Use of a silica abrasive such as precipitated silica, silica gel, silicic anhydride, aluminosilicate or gluconosilicate, secondary potassium phosphate·dihydrate or anhydrate, calcium pyrophosphate, calcium carbonate, alumina, aluminum hydroxide, magnesium acetate, tertiary magnesium phosphate, zeolite and synthetic resin abrasives is preferred. They may be used either singly or in combination of two or more of them.

The abrasive power (RDA: Radio-active Dentin Abrasion) of an abrasive is preferably from 20 to 200, more preferably from 60 to 150 in order to prevent dentin abrasion of teeth. Use of an abrasive having an RDA of from 60 to 150 in combination with an abrasive having an RDA having 150 or greater but not greater than 200 enables to reduce the amount of the former abrasive. In this case, the abrasive having an RDA of from 60 to 150 to the abrasive having an RDA of 150 or greater but not greater than 200 are mixed at a ratio (mass ratio) of from 1:1 to 10:1, more preferably from 2:1 to 8:1.

Here, RDA of a 10% abrasive slurry measured in accordance with the method of Hefferen is used (J. Dent. Res. 55, 563(1976).

The content of the abrasive(s) is from 10 to 30 weight%, preferably from 12 to 25 weight%, more preferably from 14 to 20 weight% based on the whole dentifrice composition.

With regard to a ratio of the abrasive to the cellulose powder in the dentifrice composition of the present invention, the content of the abrasive is preferably from 10 to 50 parts by mass, more preferably from 25 to 40 parts by mass when the content of the cellulose powder is 1 part by mass.

The dentifrice composition of the present invention preferably contains (D) granules further. The granules (D) to be used in the present invention have a particle size (from 75 to 500 $\mu$m) which passes through a 30-mesh sieve (JIS standards) but does not pass through a 200-mesh sieve (JIS standards). Examples of such granules include granules available by binding water insoluble powder materials with a water insoluble inorganic binder as described in JP-B-06-021053, granules available by coagulating only fine particles of calcium carbonate as described in Japanese Patent No. 3170250, granules available by binding water insoluble powder materials with a water insoluble organic binder as described in JP-04-243815, and wet-method silica granules having a specific surface area, as measured by the BET method, of from 150 to 450 m$^2$/g as described in JP-09-12436. Those having a disintegration strength when 0.1 to 30 g of a load is applied per granule are preferred from the viewpoint of feeling upon use. The content of the granules is preferably from 0.5 to 30 weight%, more preferably from 1 to 30 weight%, still more preferably from 1 to 25 weight%, still more preferably from 2 to 20 weight% in the whole dentifrice composition.

The dentifrice composition of the present invention may contain a polyphosphate further. Examples of the polyphosphate include linear polyphosphates such as sodium pyrophosphate, potassium pyrophosphate, sodium tripolyphosphate, potassium tripolyphosphate, sodium tetapolyphosphate, potassium tetapolyphosphate and sodium metaphosphate and cyclic polyphosphates such as sodium trimetaphosphate, potassium trimetaphosphate, sodium tetrametaphosphate, potassium tetrametaphosphate, sodium hexametaphosphate and potassium hexametaphosphate. These polyphosphates may be used either singly or as a mixture of two or ore of them. Of these, linear polyphosphates are preferred, with those having a polymerization of from 2 to 4 being especially preferred. The content of the polyphosphate is preferably from 0.05 to 10 weight%, more preferably from 0.1 to 8 weight%, even more preferably from 0.1 to 5 weight%.

The dentifrice composition of the present invention may further contain polyethylene glycol. The polyethylene glycol to be incorporated in the dentifrice composition of the present invention has preferably an average molecular weight of from 200 to 1000 and its content is preferably from 1 to 10 weight%, more preferably from 2 to 8 weight%, even more preferably from 3 to 7 weight% in the whole composition.

The dentifrice composition of the present invention preferably further contains a binder. It is preferred to add one or more binders selected from the group consisting of sodium alginate, sodium carboxymethyl cellulose, carrageenan, xanthan gum, sodium polyacrylate, hydroxyethyl cellulose, hydroxypropyl cellulose, pectin, tragacanth gum, arabic gum, guar gum, karaya gum, locust bean gum, gellan gum, tamarind gum, Psyllium seed gum, polyvinyl alcohol, sodium chondroitin sulfate, and methoxyethylene maleic anhydride copolymer. Of these, sodium alginate, carboxymethyl cellulose, carrageenan, and xanthan gum are especially preferred. The content of the binder is preferably from about 0.1 to 3 weight%, more preferably from 0.2 to 2 weight%, still more preferably from 0.2 to 1.5, even more preferably from 0.5 to 1.5 weight% in the whole dentifrice composition.

The dentifrice composition of the present invention can contain, in addition to the above-described components, for example, a foaming agent, a foaming assistant, an abrasive, a humectant, a sweetener, a preservative, an enzyme, a pH regulator, a bactericide, a pharmaceutically effective component, a pigment, a colorant and flavor as needed.

Preferred examples of the humectant include glycerin, sorbitol, propylene glycol, polyethylene glycol, xylitol, maltitol, lactitol and trehalose. The content of the humectant, if any, is preferably fro 1 to 10 weight%, more preferably from 2 to 8 weight%, even more preferably from 3 to 7 weight% in the whole dentifrice composition.

Examples of the sweetener include saccharin sodium, aspartame, thaumatin, acesulfame potassium, stevioside, stevia extract, paramethoxy cinnamic aldehyde, neohesperidin dihydrochalcon and perillartine.

Examples of the pH regulator include phosphoric acid and salts thereof (such as sodium phosphate and sodium hydrogen phosphate), citric acid and salts thereof (such as sodium citrate), malic acid and salts thereof, gluconic acid and salts thereof, maleic acid and salts thereof, aspartic acid and salts thereof, succinic acid and salts thereof, glucuronic acid and salts thereof, fumaric acid and salts thereof, glutamic acid and salts thereof, adipic acid and salts thereof, hydrochloric acid, sodium hydroxide, potassium hydroxide and sodium silicate. Although there is no particular limitation imposed on the content of the pH regulator insofar as the composition has a desired pH, it is usually added in an amount of from about from 0.01 to 5 weight%, preferably from about 0.1 to 3 weight% based on the whole composition. Although no particular limitation is imposed on the pH of the dentifrice composition of the present invention insofar as it can exhibit the advantages.of the present invention, it is usually from about 4 to 10.

Examples of the flavor include 1-menthol, carvone, anethole, eugenol, limonene, peppermint oil, spearmint oil, ocimene, n-amyl alcohol, citronellol, $\alpha$-terpineol, methyl salicylate, methyl acetate, acetate, cineol, linalool, ethyl linalool, vanillin, thymol, lemon oil, orange oil, sage oil, rosemary oil, cinnamon oil, pimento oil, perilla oil, clove oil and eucalyptus oil.

Examples of the other various medicinally effective ingredients include water-soluble phosphoric acid compounds such as potassium salt or sodium salt of orthophosphoric acid, allantoin chlorohydroxyaluminum, hinokitiol, lysozyme chloride, glycyrrhizinic acid and salts thereof, sodium chloride, tranexamic acid, epsilon-aminocaproic acid, dl-tocopherol acetate, azulene, glycyrrhetinic acid, copper compounds such as sodium copper chlorophyllin and copper gluconate, aluminum lactate, strontium chloride, potassium nitrate, berberine, hydroxamic acid and derivatives thereof, sodium tripolyphosphate, zeolite, dextranase, mutanase, amylase, methoxyethylene, maleic anhydride copolymer, polyvinylpyrrolidone, epidihydrocholesterin, dihydrocholesterol, zinc citrate, extracts of Japanese angelica roots, phellodendron barks, clove, rosemary, scutellaria roots, safflower, and the like, benzethonium chloride, trichlorocarbanilide, $\alpha$-bisabolol, chlorhexidine salts, triclosan, cetylpyridinium chloride, benzethonium chloride, and trichlorocarbanilide.

The water content can be determined as needed depending on the form of the composition, but it is usually from about 0 to 60 weight%, preferably from about 10 to 50 weight% based on the whole dentifrice composition.

[Examples]

The present invention will hereinafter be described in full detail by Examples and Comparative Examples. It should however be borne in mind that the present invention is not limited to or by these Examples. All designations of "%" mean weight% unless otherwise specifically indicated.

Examples 1 to 5 and Comparative Examples 1 and 2

Toothpastes were prepared in accordance with the formulations as described in Table 1.

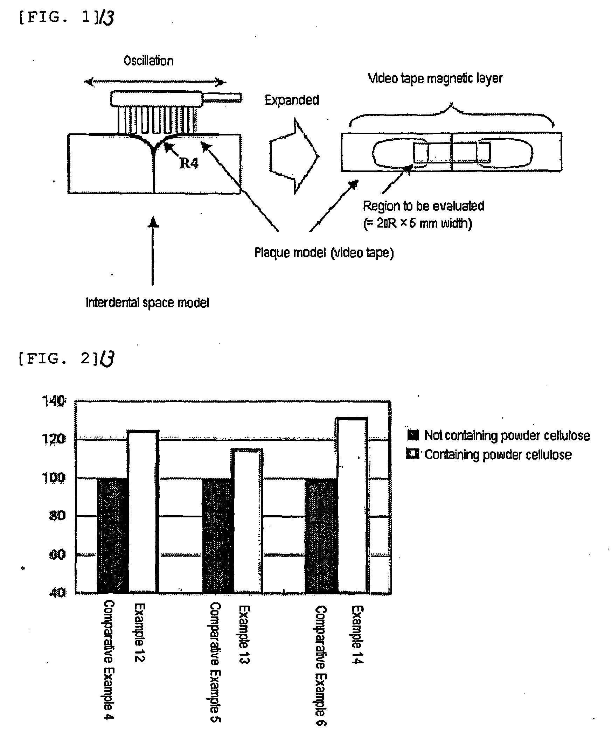

The dentifrice compositions thus obtained was tested to know their stain removing effect at the interdental space. Described specifically, as illustrated in FIG. 1, an interdental space model was formed using aluminum blocks while facing their R4 curved surfaces each other. A video tape magnetic layer was attached as a colored model to the surface of the resulting interdental space model. By using a brushing machine as illustrated in FIG. 2, the interdental model thus formed was cleaned with each of the toothpastes obtained in Examples 1 to 5 and those obtained in Comparative Examples 1 and 2. A toothbrush ("Check Standard", product of Kao) was used for brushing under the following conditions: load of 300 g, rate of 120 r/min, amplitude of 30 mm and 120 brushing times. A region of $2\pi R4 \times 5$ mm width was evaluated as an interdental space. After cleaning by brushing, the video tape magnetic layer was expanded, photographed by a digital camera and subjected to image analysis. An area (mm$^2$) of a portion which had become white because the magnetic layer was peeled from the evaluation region was calculated by the image analysis and the whitening and stain removal ratio at the interdental space was evaluated. The evaluation results are shown in Table 1. With the whitening and stain removal effects at the interdental space in Comparative Example 1 as a reference (100%), a removal ratio relative thereto was indicated as evaluation results.

[Table 1]

| (weight%) | Examples | | | | | Comparative Examples | |
|---|---|---|---|---|---|---|---|
| | 1 | 2 | 3 | 4 | 5 | 1 | 2 |
| Abrasive silica *1 | 20.00 | 20.00 | 20.00 | 20.00 | 19.00 | 20.00 | 20.00 |
| Powder cellulose (average particle size: 20μm) | 0.50 | | | | 2.00 | | |
| Powder cellulose (average particle size: 30μm) | | 0.50 | | | | | |
| Powder cellulose (average particle size: 40μm) | | | 0.50 | | | | |
| Powder cellulose (average particle size: 50μm) | | | | 0.50 | | | |
| Crystalline cellulose (average particle size: 10 μm or less) | | | | | | | 0.50 |
| Polyethylene glycol(PEG600) | 5.00 | 5.00 | 5.00 | 5.00 | 5.00 | 5.00 | 5.00 |
| Sodium lauryl sulfate | 1.50 | 1.50 | 1.50 | 1.50 | 1.50 | 1.50 | 1.50 |
| Viscous silica | 3.00 | 3.00 | 3.00 | 3.00 | 3.00 | 3.00 | 3.00 |
| Xanthan qum | 0.50 | 0.50 | 0.50 | 0.50 | 0.50 | 0.50 | 0.50 |
| Carrageenan | 0.50 | 0.50 | 0.50 | 0.50 | 0.50 | 0.50 | 0.50 |
| Concentrated glycerin | 20.00 | 20.00 | 20.00 | 20.00 | 20.00 | 20.00 | 20.00 |
| Sorbitol | 30.00 | 30.00 | 30.00 | 30.00 | 30.00 | 30.00 | 30.00 |
| Saccharin sodium | 0.15 | 0.15 | 0.15 | 0.15 | 0.15 | 0.15 | 0.15 |
| Sodium fluoride | 0.20 | 0.20 | 0.20 | 0.20 | 0.20 | 0.20 | 0.20 |
| DL-malic acid | 2.00 | 2.00 | 2.00 | 2.00 | 2.00 | 2.00 | 2.00 |
| Potassium hydroxide solution (48wt.%) | 2.50 | 2.50 | 2.50 | 2.50 | 2.50 | 2.50 | 2.50 |
| Sodium hydroxide solution (48 wt.%) | 0.50 | 0.50 | 0.50 | 0.50 | 0.50 | 0.50 | 0.50 |
| Titanium oxide | 0.30 | 0.30 | 0.30 | 0.30 | 0.30 | 0.30 | 0.30 |
| Flavor | 1.00 | 1.00 | 1.00 | 1.00 | 1.00 | 1.00 | 1.00 |
| Purified water | Balance | Balance | Balance | Balance | Balance | Balance | Balance |
| | 100 | 100 | 100 | 100 | 100 | 100 | 100 |
| Stain removing effect at the interdental space (%) | 130 | 128 | 131 | 135 | 129 | 100 | 113 |
| *1:RDA(110-150) | | | | | | | |

As is apparent from Table 1, the compositions of Examples 1 to 5 containing powder cellulose showed a superior stain removing effect at the interdental space to the composition of Comparative Example 1 not containing powder cellulose. The composition of Comparative Example 2 containing crystalline cellulose had improved the stain removing effect at the interdental space, but the effect was inferior to that of the composition containing powder cellulose.

Examples 6 to 11 and Comparative Example 3

Oral compositions were prepared in accordance with the formulation shown in Table 2.

[Table 2]

| | | | | | | | (weight%) |
|---|---|---|---|---|---|---|---|
| (weight%) | Examples | | | | | | Comp. Ex. |
| | 6 | 7 | 8 | 9 | 10 | 11 | 3 |
| Aluminum hydroxide | 20.00 | 20.00 | | | | | |
| Alumina | | 5.00 | | | | | |
| Potassium hydrogen phosphate dihyrate | | | | 30.00 | | 20.00 | |
| Anhydrous potassium hydrogen phosphate | | | | | | 10.00 | |
| Abrasive silica (RDA:150~200) | | | | | 3.00 | | |
| Abrasive silica (RDA:110~150) | 16.00 | | | | 13.00 | | 3.00 |
| Abrasive silica (RDA:値20~110) | | | 25.00 | | | | |
| Powder cellulose (average particle size: 20$\mu$m) | 0.50 | 0.50 | 0.50 | | 0.30 | | |
| Powder cellulose (average particle size: 50$\mu$m) | | | | 0.20 | | 1.00 | |
| Powder cellulose | | | | | | | 20.0 |
| Polyethylene glycol(PEG600) | 5.00 | | 5.00 | | | | 5.00 |
| Sodium lauryl sulfate | 1.50 | 1.50 | | 1.60 | 1.00 | 1.60 | 1.50 |
| Sodium N-lauroyl-L-glutamate | | | | | | 0.30 | |
| POE (20) sorbitan monostearate | | | 0.30 | | | | |
| POE(60) hydrogenated castor oil | | | | | | 1.00 | |
| Glycerin fatty acid ester | | | 1.50 | | | | |
| Thickening silica | 4.00 | 4.00 | 4.00 | 1.00 | 5.00 | | |
| Sodium polyacrylate | | | | | | 0.50 | |
| Xanthan gum | 0.20 | 0.30 | 0.30 | | 0.30 | | 0.50 |
| Carboxymethylcellulose sodium | | 0.50 | | 0.30 | | 0.30 | |
| Carrageenan | 0.60 | | | 0.20 | 0.30 | | 0.50 |
| Sodium alginate | | | 0.50 | | | | |
| Propylene glycol | | | | | 4.00 | | |
| Concentrated glycerin | 15.00 | 30.00 | 15.00 | 15.00 | 20.00 | 25.00 | 25.00 |

(continued)

| (weight%) | Examples | | | | | | Comp. Ex. |
|---|---|---|---|---|---|---|---|
| | 6 | 7 | 8 | 9 | 10 | 11 | 3 |
| Sorbitol | 30.00 | 15.00 | 20.00 | 30.00 | 30.00 | 25.00 | 23.00 |
| Saccharin sodium | 0.15 | 1.30 | 0.15 | 0.12 | 0.15 | 0.12 | 0.12 |
| Sodium fluoride | 0.20 | | 0.20 | | 0.20 | | 0.20 |
| Sodium monofluorophosphate | | 0.70 | | 0.70 | | 0.70 | |
| dl-α-tocopherol acetate | | | | | 0.30 | | |
| DL-malic acid | 2.00 | 2.00 | 2.20 | | | | 2.00 |
| Potassium hydroxide solution (48 weight%) | | | 2.50 | | | | |
| Sodium hydroxide solution (48 weight%) | 2.50 | 2.50 | | | | | 2.50 |
| L-arginine | | | 1.20 | | | | |
| Triclosan | | 0.10 | | | | 0.10 | |
| Benzethonium chloride | | | | 0.01 | | | |
| Titanium oxide | 0.30 | | 0.30 | 0.30 | 0.30 | | 0.30 |
| Zinc chloride | | | | 0.50 | | | |
| Flavor | 1.00 | 1.00 | 1.00 | 1.00 | 1.00 | 1.00 | 1.00 |
| Purified water | Balance | Balance | Balance | Balance | Balance | Balance | Balance |
| | 100 | 100 | 100 | 100 | 100 | 100 | 100 |

(weight%)

The dentifrice compositions of the present invention obtained in Examples 6 to 11 exhibited, similar to those obtained in Examples 1 to 5, an excellent stain removing effect at the interdental space, but the composition obtained in Comparative Example 3 exhibited a. stain removing effect as low as 0.3%.

Examples 12 to 14 and Comparative Examples 3 to 5

Toothpastes were prepared in accordance with the formulations as described in Table 3.

[Table 3]

| (weight%) | Ex. 12 | Comp. Ex. 4 | Ex. 13 | Comp. Ex. 5 | Ex. 14 | Comp. Ex. 6 |
|---|---|---|---|---|---|---|
| Abrasive silica (RDA:110-150) | 16.00 | 16.00 | 16.00 | 16.00 | 16.00 | 16.00 |
| Powder cellulose (average particle size: 50μm) | 0.50 | | 0.50 | | 0.50 | |
| Silica granules (average particle size: 200μm) | 3.00 | 3.00 | | | | |
| Sodium tripolyphosphate (STPP) | | | 0.10 | 0.10 | | |
| Polyethylene glycol (PEG600) | | | | | 5.00 | 5.00 |
| Sodium lauryl sulfate | 1.50 | 1.50 | 1.50 | 1.50 | 1.50 | 1.50 |

(continued)

| | Ex. 12 | Comp. Ex. 4 | Ex. 13 | Comp. Ex. 5 | Ex. 14 | Comp. Ex. 6 |
|---|---|---|---|---|---|---|
| | | | | | | (weight%) |
| Thickening silica | 4.00 | 4.00 | 4.00 | 4.00 | 4.00 | 4.00 |
| Xanthan gum | 0.20 | 0.20 | 0.20 | 0.20 | 0.20 | 0.20 |
| Carrageenan | 0.60 | 0.60 | 0.60 | 0.60 | 0.60 | 0.60 |
| Propylene glycol | 5.00 | 5.00 | 5.00 | 5.00 | | |
| Concentrated glycerin | 20.00 | 20.00 | 20.00 | 20.00 | 20.00 | 20.00 |
| Sorbitol | 30.00 | 30.00 | 30.00 | 30.00 | 30.00 | 30.00 |
| Saccharin sodium | 0.15 | 0.15 | 0.15 | 0.15 | 0.15 | 0.15 |
| Sodium fluoride | 0.20 | 0.20 | 0.20 | 0.20 | 0.20 | 0.20 |
| DL-Malic acid | 2.00 | 2.00 | 2.00 | 2.00 | 2.00 | 2.00 |
| Sodium hydroxide solution (48 weight%) | 2.50 | 2.50 | 2.50 | 2.50 | 2.50 | 2.50 |
| Titanium oxide | 0.30 | 0.30 | 0.30 | 0.30 | 0.30 | 0.30 |
| Flavor | 1.00 | 1.00 | 1.00 | 1.00 | 1.00 | 1.00 |
| Purified water | Balance | Balance | Balance | Balance | Balance | Balance |
| | 100 | 100 | 100 | 100 | 100 | 100 |

In a similar manner to Example 1, the toothpastes thus obtained were tested for the stain removing effect at the interdental space. The evaluation results are shown in FIG. 2. With the stain removing effect at the interdental space in Comparative Examples 3 to 5 corresponding to Examples 12 to 14, respectively as a reference (100%), the evaluation results of them were indicated by a relative removing ratio.

As is apparent from FIG. 2, the toothpastes of Examples 12 to 14 containing powder cellulose were superior in stain removing effect at the interdental space to those of Comparative Examples 3 to 5 not containing powder cellulose.

Example 15

Toothpastes were prepared in accordance with the formulations as shown in Table 4.

[Table 4]

| | Example 15 | Comp. Ex. 7 | Comp. Ex.8 |
|---|---|---|---|
| Calcium carbonate | | 50.00 | 5.00 |
| Silicic anhydride | 18.50 | | 7.00 |
| Powder cellulose (average particle size: about 50 $\mu$m) | 0.50 | | |
| Polyethylene glycol (PEG600) | 5.00 | | 5.00 |
| Silicic anhydride granules (average particle size: 200 $\mu$m) | 2.50 | | 14.00 |
| Sodium lauryl sulfate | 1.50 | 1.50 | 1.50 |
| Xanthan gum | 0.40 | | 0.20 |
| Carrageenan | 0.60 | 0.50 | |
| Carboxylmethylcellulose sodium | | 1.00 | 1.50 |
| Concentrated glycerin | 20.00 | 4.00 | |
| Sorbitol (sorbitol solution) | 30.00 | 15.00 | 36.00 |
| Saccharin sodium | 0.15 | 0.15 | 0.15 |

(continued)

|  | Example 15 | Comp. Ex. 7 | Comp. Ex.8 |
|---|---|---|---|
| Sodium fluoride | 0.20 | | |
| Sodium monofluorophosphate | | 0.70 | 0.70 |
| DL-Malic acid | 2.00 | | |
| pH Repulator | Amount to adjust pH to 6.0 | | |
| Flavor | 1.40 | 0.85 | 1.00 |
| Purified water | Balance | Balance | Balance |
| Total | 100 | 100 | 100 |

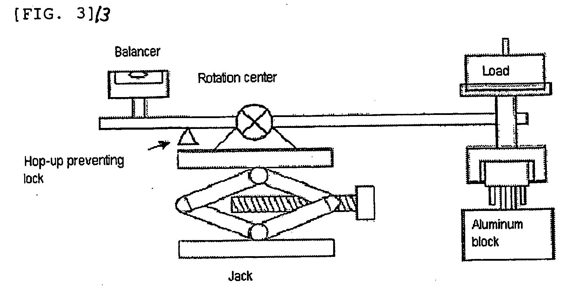

The dentifrice compositions thus obtained were tested for their foaming property. A surface to be brushed was formed by arranging, on an acrylic plate (30 × 80 × 3 mm), columnar-shaped acrylic rods having $\varphi$3 vertically relative to the pedestal running direction. An acrylic vessel (80 × 175 × 35 mm) was attached to fix the surface. The toothpastes of Example 15 and toothpastes of Comparative Examples 6 and 7 were each diluted to 5 times the original weight and a foaming test of the diluted toothpaste was performed by moving a brushing machine (FIG. 3, having a horizontally movable pedestal) on the surface to be brushed.

Cleaning by brushing was conducted by using a toothbrush ("Kesakigakyu", trade name; product of Kao) under a load of 500 g, rate of 120 r/min, amplitude of 50 mm and cleaning of 500 times. The liquid remaining after cleaning was transferred to a measuring cylinder and the foam amount was measured. The amount (mL) of foams present in the upper part of the measuring cylinder is shown in Table 5 as the evaluation results of the foaming property.

[Table 5]

| Foam volume (mL) | Time after tooth brushing (minute) | |
|---|---|---|
| | 1 | 15 |
| Example 15 | 61 | 60 |
| Comp. Ex. 7 | 56 | 52.5 |
| Comp. Ex. 8 | 54 | 52 |

A panel of five experts was asked to use each toothpaste and organoleptically evaluate the foam quality at the time of using from the viewpoints of texture of foam, wateriness of foam, viscosity of foam and foaming ease based on the below-described criteria.

(1) Texture of foam

Fine: 2
Rather fine 1:

Difficult to evaluate: 0
Rather coarse: -1

Coarse: 1

(2) Elasticity of foam

Elastic: 2
Rather elastic: 1
Difficult to evaluate: 0
Slightly watery: -1
Watery: -2

(3) Viscosity of foam

Viscous: 2
Rather viscous: 1
Difficult to evaluate: 0
Slightly viscous: -1
Not viscous: -2

(4) Foaming property at the time of use

Prompt: 2
Rather prompt: 1
Difficult to evaluate: 0
Rather slow: -1
Slow: -2

The total evaluation scores of five experts are shown in Table 6.

[Table 6]

|  | Texture of foam | Wateriness of foam | Viscosity of foam | Foaming property |
| --- | --- | --- | --- | --- |
| Example 15 | 5 | 6 | 3 |  |
| Comparative Example 7 | -1 | 4 | 1 | 2 |
| Comparative Example 8 | 0 | -2 | -2 | 1 |

It has been understood that the toothpaste of the present invention (Example 15) had a good foaming property and good foam quality with fine texture. The toothpaste containing neither powder cellulose nor cellulose granules (Comparative Example 7) and the toothpaste not containing powder cellulose (Comparative Example 8) were poor in both foaming property and foam quality.

**Claims**

1. A dentifrice composition comprising the following components (A), (B) and (C):

    (A) from 0.2 to 3 weight% of powder cellulose;
    (B) a surfactant; and
    (C) from 10 to 30 weight% of an abrasive,

    wherein the abrasive has an RDA of from 20 to 200.

2. The dentifrice composition according to Claim 1, wherein the content of the powder cellulose is from 0.4 to 2 weight%.

3. The dentifrice composition according to any one of Claims 1 to 2, wherein the powder cellulose has an average polymerization degree of from 350 to 2250.

4. The dentifrice composition according to any one of Claims 1 to 3, wherein the powder cellulose is a non-granulated powder cellulose having a particle size of from 10 to 300 $\mu$m.

5. The dentifrice composition according to any one of Claims 1 to 4, further comprising a binder.

6. The dentifrice composition according to Claim 5, wherein the binder comprises at least two binders selected from the group consisting of sodium alginate, sodium carboxymethyl cellulose, carrageenan, xanthan gum, sodium polyacrylate, hydroxyethyl cellulose, hydroxypropyl cellulose, pectin, tragacanth gum, arabic gum, guar gum, karaya gum, locust bean gum, gellan gum, tamarind gum, Psyllium seed gum, polyvinyl alcohol, sodium chondroitin sulfate, and methoxyethylene maleic anhydride copolymer.

**Patentansprüche**

1. Zahnputzmittelzusammensetzung, umfassend die folgenden Komponenten (A), (B) und (C):

   (A) 0,2 bis 3 Gew-% Pulvercellulose;
   (B) ein Tensid; und
   (C) 10 bis 30 Gew-% eines Abrasivstoffs,

   wobei der Abrasivstoff einen RDA von 20 bis 200 aufweist.

2. Zahnputzmittelzusammensetzung gemäß Anspruch 1, wobei der Gehalt der Pulvercellulose 0,4 bis 2 Gew-% beträgt.

3. Zahnputzmittelzusammensetzung gemäß einem der Ansprüche 1 bis 2, wobei die Pulvercellulose einen durchschnittlichen Polymerisationsgrad von 350 bis 2250 aufweist.

4. Zahnputzmittelzusammensetzung gemäß einem der Ansprüche 1 bis 3, wobei die Pulvercellulose eine nichtgranulierte Pulvercellulose mit einer Partikelgröße von 10 bis 300 $\mu$m ist.

5. Zahnputzmittelzusammensetzung gemäß einem der Ansprüche 1 bis 4, zusätzlich umfassend ein Bindemittel.

6. Zahnputzmittelzusammensetzung gemäß Anspruch 5, wobei das Bindemittel mindestens zwei Bindemittel umfasst, die aus der Gruppe ausgewählt sind, die aus Natriumalginat, Natriumcarboxymethylcellulose, Carrageenan, Xanthangummi, Natriumpolyacrylat, Hydroxyethylcellulose, Hydroxypropylcellulose, Pektin, Tragantgummi, Gummi arabicum, Guargummi, Karayagummi, Johannisbrotgummi, Gellangummi, Tamarindengummi, Psylliumsamengummi, Polyvinylalkohol, Natriumchondroitinsulfat und Methoxyethylen-Maleinsäureanhydrid-Copolymer besteht.

**Revendications**

1. Composition de dentifrice comprenant les composés (A), (B) et (C) suivants :

   (A) de 0,2 à 3 % en poids de cellulose en poudre ;
   (B) un surfactant; et
   (C) de 10 à 30 % en poids d'un abrasif,

   dans laquelle l'abrasif a un indice RDA de 20 à 200.

2. Composition de dentifrice selon la revendication 1, dans laquelle la teneur en cellulose en poudre est de 0,4 à 2 % en poids.

3. Composition de dentifrice selon l'une quelconque des revendications 1 à 2, dans laquelle la cellulose en poudre a un degré moyen de polymérisation de 350 à 2250.

4. Composition de dentifrice selon l'une quelconque des revendications 1 à 3, dans laquelle la cellulose en poudre est une cellulose en poudre non granulée ayant une taille de particule de 10 à 300 $\mu$m.

5. Composition de dentifrice selon l'une quelconque des revendications 1 à 4, comprenant en outre un liant.

6. Composition de dentifrice selon la revendication 5, dans laquelle le liant comprend au moins deux liants sélectionnés dans le groupe constitué d'alginate de sodium, de carboxyméthylecellulose de sodium, de carraghénane, de gomme de xanthane, de polyacrylate de sodium, d'hydroxyéthylcellulose, d'hydroxypropylcellulose, de pectine, de gomme adragante, de gomme arabique, de gomme de guar, de gomme karaya, de gomme de caroube, de gomme de gellane, de gomme de tamarin, de gomme de graines de Psyllium, d'alcool polyvinylique, de chondroïtine sulfate de sodium, et d'un copolymère de méthoxyéthylène-anhydride maléique.

[FIG. 1]1/3

Oscillation

Video tape magnetic layer

Expanded

R4

Region to be evaluated
(= 2πR × 5 mm width)

Plaque model (video tape)

Interdental space model

[FIG. 2]1/3

■ Not containing powder cellulose
□ Containing powder cellulose

Comparative Example 4 · Example 12 · Comparative Example 5 · Example 13 · Comparative Example 6 · Example 14

[FIG. 3]/3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 55098111 A
- JP 9040537 A
- JP 11209255 A
- WO 2004071321 A2
- JP 11199456 A
- EP 0480172 A1
- JP 6021053 B
- JP 3170250 B
- JP 4243815 A
- JP 9012436 A